# EUROPEAN PATENT APPLICATION

(11) **EP 4 257 695 A1**
(43) Date of publication of application: **11.10.2023**
(21) Application number: 21902567.3
(22) Date of filing: 06.12.2021
(51) Int. Cl.: C12N 15/867, C12N 5/10

(54) **LENTIVIRUS PACKAGING VECTOR AND PACKAGING METHOD THEREOF**

(30) Priority: 07.12.2020 CN 202011419453
(71) Applicant: Obio Technology (Shanghai) Corp., Ltd., Shanghai 200135 (CN)
(72) Inventor: YANG, Xinglin, Shanghai 200135 (CN); YOU, Qingrui, Shanghai 200135 (CN); YANG, Jiali, Shanghai 200135 (CN); MA, Peimin, Shanghai 200135 (CN); JIA, Guodong, Shanghai 200135 (CN); PAN, Oudong, Shanghai 200135 (CN)
(74) Representative: J A Kemp LLP
(86) International application number: PCT/CN2021/135859
(87) International publication number: WO 2022/121862

(57) **Abstract**

Provided are a lentivirus packaging vector and a packaging method thereof. The lentivirus packaging vector contains a first LTR, a reversely inserted gene expression cassette and a second LTR, wherein the first LTR is positioned upstream of the reversely inserted gene expression cassette in the direction of viral genome expression; the second LTR is positioned downstream of the reversely inserted gene expression cassette in the direction of viral genome expression; the gene expression cassette contains a promoter, a repressible operon, a gene of interest and a polyadenylation signal, which are connected in sequence; and in the presence of a repressor, the repressible operon represses the expression of the gene of interest.

## Description

### CROSS-REFERENCE TO RELATED APPLICATIONS

This application claims priority of Chinese Patent Application No. 202011419453.X, filed with the Chinese Patent Office on December 7, 2020, entitled "Lentivirus packaging vector and packaging method thereof, the content of which is hereby incorporated by reference in its entirety.

### TECHNICAL FIELD

The present disclosure relates to the field of biotechnology, in particular, to a vector for lentiviral packaging and a method for packaging the same.

### BACKGROUND

Lentivirus vector is a kind of viral vector engineered from human immunodeficiency virus (HIV). Lentivirus, belonging to retroviruses, has an RNA genome whose toxic gene has been deleted and replaced by an exogenous gene of interest, and thus belongs to pseudotyped viruses. By using reverse transcriptase, the exogenous gene can be integrated into the genome and stably expressed. Lentivirus has the properties of infecting both dividing cells and non-dividing cells.

After entering a cell, the lentiviral genome is reverse-transcribed into DNA which forms a DNA pre-integration complex in the cytoplasm. After entering the nucleus, the DNA is integrated into the cell genome. The integrated DNA is transcribed into mRNA, which returns to the cytoplasm and expresses the protein of interest; or produces small RNA. The expression of genes and the interference effect of small RNA which are mediated by lentivirus are sustained and stable, and the gene and small RNA replicate as the cellular genome replicates.

Current studies of lentiviral vectors focus on aspects of infectivity, specificity, packaging capacity or the like, while few attentions have been paid to effects of gene of interest (GOI) packaged on the virus. In practice, a considerable proportion of genes of interest result in not being able to obtain effective lentivirus particles due to interference with lentivirus packaging by expression of the gene of interest itself. Besides, for the lentiviral vector, the virus packaging will go through a transcription process, thus an exogenous gene containing important splicing sites such as circular RNA (circRNA) will be subjected to large-scale splicing during the packaging, which leads to the deletion of the exogenous gene sequence in the final virus particle.

Common lentiviral vectors are not suitable for expressing genes whose expression needs to be precisely terminated. For example, generally speaking, a forward polyA signal cannot be present in a lentiviral vector, because the polyA signal could cause premature termination of the viral RNA transcription, thereby seriously affecting the titer of virus. For another example, in the case of expression of long-chain non-coding RNA (LncRNA) promoted by Pol II promoter, the LncRNA will carry a large segment of vector sequence (such as, woodchuck hepatitis virus post-transcriptional regulatory element (WPRE)) due to failure to terminate the expression in time, thereby affecting the biological function of LncRNA. For still another example, in the case where an exogenous gene containing important splicing sites such as circRNA is constructed into lentiviral vectors, splicing will occur in the production of viral RNA and lead to the deletion of the exogenous gene.

It was found that a reverse expression cassette can avoid the premature termination of the viral RNA transcription caused by poly A signal, and can effectively terminate LncRNA. Also, for circRNA, the reverse expression cassette can also disable the splicing site to ensure the integrity of gene. However, the packaging of a gene whose expression needs to be precisely terminated into the lentivirus cannot be effectively achieved because there is a conflict between the reverse expression cassette and the viral RNA transcription, possibly between the transcription process of the promoter of the reverse expression cassette and the viral RNA transcription, or because the reverse transcribed RNA and the forward transcribed RNA may form a double-stranded RNA, which affects the packaging of the virus.

### SUMMARY

Various exemplary embodiments disclosed in the present disclosure provide a vector for lentiviral packaging, including a first long terminal repeat, a reversely inserted gene expression cassette, and a second long terminal repeat. The first long terminal repeat is positioned upstream of the reversely inserted gene expression cassette in a direction of viral genome expression. The second long terminal repeat is positioned downstream of the reversely inserted gene expression cassette in a direction of viral genome expression. The reversely inserted gene expression cassette includes a promoter, a repressible operon, a gene of interest, and a polyadenylation signal which are linked in sequence. The repressible operon represses expression of the gene of interest in the presence of a repressor. The gene of interest is a gene whose expression needs to be precisely terminated, or a gene that is not suitable for positioning in a forward orientation in lentiviral vectors.

According to another aspect, the present disclosure relates to a lentiviral vector packaging system, including the vector for lentiviral packaging as described above. The lentiviral vector packaging system is capable of producing HIV vector particles having only primary infectivity and no replication capacity.

According to yet another aspect, the present disclosure relates to a method for packaging a lentivirus, including transferring the lentiviral vector packaging system as described above into a host cell, and performing packaging in the presence of a repressor.

In the present disclosure, a repressible operon regulatory system is creatively applied on a reverse polyA lentiviral vector, leading to efficient packaging of the lentiviral virus including a reverse expression cassette.

### BRIEF DESCRIPTION OF THE DRAWINGS

In order to more clearly illustrate the technical solutions in the specific embodiments or some techniques of the present disclosure, the accompanying drawings needed to be used in the description of specific embodiments or some techniques are briefly described in the below. It is apparent that the accompanying drawings as described below show some embodiments of the present disclosure, and other drawings can also be obtained based on these drawings by those of ordinary skill in the art, without creative work.
Figures 1 to 5 show the plasmid maps of vectors pcDNA3.1-CymR, CMV-CuO, EF1a-CuO, SFH-CuO and CAG-CuO, respectively, in an embodiment of the present disclosure.
Figure 6 shows the results of verification of repression efficiency of CymR-CuO in a lentiviral vector in an embodiment of the present disclosure.
Figure 7 shows the plasmid map of CMV-TrpO in an embodiment of the present disclosure.
Figure 8 shows the results of verification of repression efficiency of tryptophan operon system in a lentiviral vector in an embodiment of the present disclosure.
Figure 9 shows the plasmid map of EF1a-ToxO in an embodiment of the present disclosure.
Figure 10 shows the results of verification of repression efficiency of diphtheria toxin repressor regulatory system in a lentiviral vector in an embodiment of the present disclosure.
Figure 11 shows the plasmid map of SFH-LacO in an embodiment of the present disclosure.
Figure 12 shows the result of verification of repression efficiency of lactose operon system in a lentiviral vector in an embodiment of the present disclosure.
Figure 13 is a schematic diagram showing insertion sites of TrpO element relative to TATA Box of CMV in an embodiment of the present disclosure.
Figure 14 shows the influence of different insertion sites of TrpO element on repression efficiency in an embodiment of the present disclosure.
Figure 15 is a schematic diagram showing insertion sites of CuO element relative to TATA Box of CMV in an embodiment of the present disclosure.
Figure 16 shows the influence of different insertion sites of CuO element on repression efficiency in an embodiment of the present disclosure.
Figures 17 to 19 show plasmid maps of vectors pSLenti-TK polyA-mCherry-CMV-SFH-EGFP-P2A-Puro-WPRE, pSLenti-TK polyA-mCherry-CuO-CMV-SFH-EGFP-P2A-Puro-WPRE, and pSLenti-TK polyA-mCherry-TrpO-CMV-SFH-EGFP-P2A-Puro-WPRE, respectively, in an embodiment of the present disclosure.
Figure 20 is fluorescence images showing the results of increased expression efficiency of the lentiviral vector including reverse expression cassette under the action of repressible operon in an embodiment of the present disclosure.
Figure 21 is a statistical diagram showing the results of increased titer of the lentiviral vector including reverse expression cassette under the action of repressible operon in an embodiment of the present disclosure.
Figure 22 is fluorescence images showing influence of different polyA signal sequences in the lentiviral vector including reverse expression cassette on titer in an embodiment of the present disclosure.
Figure 23 is a statistical diagram showing influence of different polyA signal sequences in the lentiviral vector including reverse expression cassette on titer in an embodiment of the present disclosure.
Figure 24 is a vector map of pSLenti-EF1a-EGFP-P2A-Puro-CMV-MCS in an embodiment of the present disclosure.
Figure 25 is a vector map of pSLenti-EF1a-EGFP-P2A-Puro-CMV-hsa_circ_0008285 in an embodiment of the present disclosure.
Figure 26 is fluorescent images showing infection or transfection of 293T cells in an embodiment of the present disclosure.
Figure 27 is a statistical diagram showing expression of hsa_circ_0008285 in an embodiment of the present disclosure.
Figure 28 is a result diagram showing expression of hsa_circ_0008285 gene from the lentiviral vector including reverse expression cassette in an embodiment of the present disclosure.
Figure 29 is a vector map of pSLenti-TK polyA-SOX2-OT-CuO-CMV-PGK-Puro-WPRE in an embodiment of the present disclosure.
Figure 30 is a vector map of pSLenti-TK polyA-SOX2-OT-TrpO-CMV-PGK-Puro-WPRE in an embodiment of the present disclosure.
Figure 31 is a result diagram showing expression of SOX2-OT gene from the lentiviral vector including reverse expression cassette in an embodiment of the present disclosure.
Figure 32 shows schematic diagrams of A) packaging of lentivirus including reverse expression cassette in the absence of repressible operon, B) packaging of lentivirus including reverse expression cassette in the presence of Cumate-CuO regulable system, and C) packaging of lentivirus including reverse expression cassette in the presence of tryptophan operon.

### DETAILED DESCRIPTION

Reference to embodiments of the present disclosure will be made in detail and one or more examples of which are described below. Each of the examples is provided by way of explanation and does not limit the present disclosure. In fact, it will be apparent to those skilled in the art that various modifications and variations can be made to the present disclosure without departing from the scope or spirit of the present disclosure. For example, features illustrated or described as a part of one embodiment is applicable to another embodiment to generate a still further embodiment.

Thus, it is intended that the present disclosure covers such modifications and variations falling within the scope of the appended claims and their equivalents. Other subjects, features and aspects of the present disclosure are disclosed in or are apparent from the following detailed description. It is understood by those skilled in the art that the present discussion is a description of exemplary embodiments only and is not intended to limit broader aspects of the present disclosure.

Unless otherwise defined, all technical and scientific terms used herein have the same meaning as commonly understood by those skilled in the technical field to which this present disclosure belongs. The terms used herein in the specification of the present disclosure are only for the purpose of describing specific embodiments and are not intended to limit the present disclosure. As used herein, the term "and/or" includes any or all combinations of one or more of the associated listed items.

The present disclosure relates to a vector for lentiviral packaging, including a first long terminal repeat, a reversely inserted gene expression cassette, and a second long terminal repeat. The first long terminal repeat is positioned upstream of the reversely inserted gene expression cassette in a direction of viral genome expression. The second long terminal repeat is positioned downstream of the reversely inserted gene expression cassette in a direction of viral genome expression. The reversely inserted gene expression cassette includes a promoter, a repressible operon, a gene of interest, and a polyadenylation signal which are linked in sequence. The repressible operon represses expression of the gene of interest in the presence of a repressor. The gene of interest is a gene whose expression needs to be precisely terminated.

Term "vector" in the present disclosure refers to a nucleic acid delivery vehicle into which a polynucleotide can be packaged. In the case where a vector enables the expression of the protein encoded by the inserted polynucleotide, the vector is called an expression vector. A vector can be introduced into a host cell by transformation, transduction or transfection, so that the genetic material element carried by the vector can be expressed in the host cell. In an embodiment, the vector is but not limited to a plasmid.

In some embodiments, the gene of interest is configured to express non-coding RNA expressed by Pol II promoter.

In some embodiments, the gene of interest is configured to express small nuclear RNA (snRNA), lncRNA, artificial microRNA (amiRNA), or circRNA.

In some embodiments, the polyadenylation signal is TK polyA and/or SV40 polyA V2.

In some embodiments, the repressible operon is selected from a tryptophan operon and/or a Cumate-CuO regulable system.

In some embodiments, the repressible operator has one or more copies.

In some embodiments, a distance between a site where a TrpO element of a tryptophan operon is inserted into the gene expression cassette and a TATA BOX of the promoter is less than or equal to 18 nucleotides, such as 17, 16, 15, 14, 13, 12, 11, 10, 9, 8, 7, 6, 5, 4, 3, 2 or 1 nucleotide(s).

In some embodiments, a distance between a site where a CuO element of a Cumate-CuO regulable system is inserted and a TATA BOX of the promoter is 40 to 50 nucleotides, such as 41, 42, 43, 44, 45, 46, 47, 48 or 49 nucleotides.

The distance between the site where the above-mentioned element of the repressible operator is inserted and the TATA BOX of promoter refers to the number of nucleotide(s) between the first nucleotide (exclusive) at the 5' end of the element and the first nucleotide (exclusive) at the 3' end of the TATA BOX.

It should be noted that the eight nucleotides (including the 8th nucleotide) following the TATA BOX are considered to belong to a core region of the promoter. In an embodiment, the distance between the site where the TrpO element is inserted and the TATA BOX of the promoter is greater than 8 nucleotides. In the case where the TrpO element has a sequence that is overlapped with said eight nucleotides, the TrpO element can be closer to the TATA BOX.

The "promoter" is a DNA sequence that directs the binding of RNA polymerase and thereby initiates RNA synthesis. As used in the present disclosure, the promoter allows expression in a wide variety of types of cells and tissues. The promoter may be a non-cell-specific promoter. Alternatively, the promoter may be a cell-specific promoter, a cell type-specific promoter, a cell lineage-specific promoter, or a tissue-specific promoter, which allow the expression in their respective species-restricted types of cells or tissues. In particular embodiments, it may be desirable to use expression control sequences specific to cells, cell types, cell lineages or tissues, to achieve the cell type-specific, cell lineage-specific or tissue-specific expression of a desired polynucleotide sequence, e.g., expression of a nucleic acid encoding a polypeptide only in a subgroup of cell types, cell lineages, or tissues, or at specific developmental stages.

Exemplary examples of tissue-specific promoters include but are not limited to, B29 promoter (expressed in B cells), runt transcription factor (CBFa2) promoter (specificly expressed in stem cells), CD14 promoter (expressed in monocytes), CD43 promoter (expressed in leukocytes and platelets), CD45 promoter (expressed in hematopoietic cells), CD68 promoter (expressed in macrophages), CYP4503A4 or ALB promoter (expressed in hepatocytes), desmin promoter (expressed in muscle cells), elastase 1 promoter (expressed in pancreatic acinar cells), endothelial glycoprotein promoter (expressed in endothelial cells), fibroblast-specific protein 1 (FSP1) promoter (expressed in fibroblasts), fibronectin promoter (expressed in fibroblasts), fms-associated tyrosine kinase 1 (FLT1) promoter (expressed in endothelial cells), glial fibrillary acidic protein (GFAP) promoter (expressed in astrocytes), insulin promoter (expressed in pancreatic cells), integrin-α-2b (ITGA2B) promoter (megakaryocytes), intracellular adhesion molecule 2 (ICAM-2) promoter (endothelial cells), interferon-β (IFN-β) promoter (hematopoietic cells), keratin 5 promoter (expressed in keratinocytes), myoglobin (MB) promoter (expressed in muscle cells), myogenic differentiation 1 (MYOD1) promoter (expressed in muscle cells), nephropathy protein promoter (expressed in podocytes), bone γ-carboxyl glutamic acid protein 2 (OG-2) promoter (expressed in osteoblasts), 3-ketoacid CoA transferase 2B (Oxct2B) promoter (expressed in haploid spermatocytes), surface activating protein B (SP-B) promoter (lung cell), synapsin promoter (expressed in neuronal cells), or Wiskott-Aldrich syndrome protein (WASP) promoter (expressed in hematopoietic cells).

In some embodiments, the promoter is a non-cell-specific promoter. Exemplary non-cell-specific promoters include but are not limited to, cytomegalovirus (CMV) immediate-early promoter, viral simian virus 40 (SV40) (e.g., early or late) promoter, Moloney murine leukemia virus (MoMLV) LTR promoter, Rous sarcoma virus (RSV) LTR promoter, herpes simplex virus (HSV) (thymidine kinase) promoter, cowpox virus H5, P7.5 and Pll promoters, elongation factor 1-alpha (EF1a) promoter, early growth response 1 (EGR1) promoter, ferritin H (FerH) promoter, ferritin L (FerL) promoter, glyceraldehyde-3-phosphate dehydrogenase (GAPDH) promoter, eukaryotic translation initiation factor 4A1 (EIF4A1) promoter, heat shock 70kDa protein 5 (HSPA5) promoter, heat shock protein 90kDa-β member 1 (HSP90B1) promoter, heat shock protein 70kDa (HSP70) promoter, β-kinesin (β-KIN) promoter, human R0SA26 locus, ubiquitin C promoter (UBC), phosphoglycerate kinase-1 (PGK) promoter, cytomegalovirus enhancer/chicken β-actin (CAG) promoter, or β-actin promoter. The non-cell-specific promoters enable better versatility and expression efficiency of the described gene expression cassettes.

In some embodiments, the promoter is a strong promoter.

In some embodiments, the promoter is a CMV, EF1a, SFH, CAG, CBh, UBC, SFFV, SV40, RSV, mCMV, GAPDH, PGK, CASI, SMVP, GUSB (hGBp), or UCOE promoter.

In some embodiments, the vector for lentiviral packaging further includes at least one of a reporter gene, an enhancer, an internal ribosome entry site, or a terminator.

According to another aspect, the present disclosure relates to a lentiviral vector packaging system, including the vector for lentiviral packaging as described above. The lentiviral vector packaging system is capable of producing HIV vector particles having only primary infectivity and no replication capacity.

In some embodiments, the lentiviral vector packaging system is a two-plasmid packaging system, a three-plasmid packaging system, or a four-plasmid packaging system.

In some embodiments, the HIV vector particles are HIV-1 or HIV-2 vector particles.

According to yet another aspect, the present disclosure relates to a method for packaging a lentivirus, including: transferring the lentiviral vector packaging system as described above into a host cell, and performing packaging in the presence of a repressor.

In some embodiments, the host cell is a mammalian cell or an avian animal cell.

In some embodiments, the host cell can also be a fish cell or an amphibian cell.

In some embodiments, the host cell is a rodent cell, such as rat, mouse, or hamster.

In some embodiments, the host cell is a primate cell. In an embodiment, the host cell is a human cell.

In some embodiments, the host cell is a primary cell, such as a tumor cell, a liver cell, a cardiomyocyte, a neuron, an endothelial cell, a stem cell, and the like.

In some embodiments, the host cell is a cell line.

Common cell lines are as follows:
human-derived cell lines:
   293, IMR-90, W1-38, A549, A431, BHL-100, BeWo, Caco-2, Chang, HCT-15, HeLa, HEp-G2, HEp-2, HT-1080, HT-29, JEG-2, MCF7, KB, Saos-2, WI-38, WISH, WS1, HUVEC, EB-3, Raji, IM-9, Daudi, H9, HL-60, Jurkat, K-562, U937, and KG-1;
mouse-derived cell lines:
   McCoy, BALB/3T3, 3T6, A9, AtT-20, Clone M-3, I-10, Y-1, WEHI-3b, ES-D3, and F9;
hamster-derived cell lines:
   BHK-21, HaK, and CHO-K1;
rat-derived cell lines:
   AR42J, BRL3A, Clone 9, H4--II-E-C3, GH1, GH3, IEC-6, L2, XC, LLC-WRC 256, Jensen, Rat2(TK-), PC12, and L6; and
cell lines derived from other animals:
   D-17, BT, MARC-145, CV-1, COS-1, COS-3, COS-7, Vero, B95-8, and CRFK.

Embodiments of the present disclosure will be described in detail below in combination with examples.

### Example 1. Packaging of lentivirus and titer test

### I. Packaging of lentivirus

A method for viral packaging using lentiviral vectors specifically includes the follows.
1. One day before transfection, 293T cells were seeded onto a culture dish. One hour before transfection, the culture dish was taken out and, with the spent cell culture medium discarded and replaced with Opti-MEM medium, placed back in the incubator. Then, a complex of transfection reagent and plasmid was prepared in the following steps.
2. Viral vector plasmids prepared for transfection (including backbone plasmids pCAG-gagpol, envelope protein plasmids pHCMV-VSVG and shuttle plasmids) were dissolved in Opti-MEM medium, gently mixed, and left standing to obtain a diluted solution of plasmid. The backbone plasmids were pCAG-gagpol, pCAG-gagpol-CMV-CymR or pCAG gagpol-CMV-TrpR. The envelope protein plasmids were pHCMV-VSVG, pHCMV-VSVG-CMV-CymR, or pHCMV-VSVG-CMV-TrpR.
3. The transfection reagent was dissolved in Opti-MEM medium, gently mixed and left standing to obtain a diluted solution of transfection reagent. The diluted solution of transfection reagent was added dropwise to the diluted solution of plasmid with gentle mixing during the addition, and then kept at room temperature for 15 to 25 mins, allowing the DNA and the transfection reagent to be thoroughly combined to form a stable transfection complex. The cell culture dish was taken out and, with the prepared complex of DNA and transfection reagent added, placed back in the incubator.
4. After 5 to 8 hours, the medium was aspirated and, after washing the dish with phosphate buffered saline (PBS), replaced with fresh complete medium for culturing. For the tryptophan operon group (i.e., pSLenti-TK PolyA-mCherry-TrpO-CMV-SFH-EGFP-P2A-Puro-WPRE), the complete medium containing 0.3 mM tryptophan was used in the replacement.
5. At 48 hours after transfection, the first virus harvest was performed by collecting the medium into a 50 ml centrifuge tube, and then the spent medium was replaced with fresh complete medium. At 72 hours after transfection, the second virus harvest was performed by collecting the medium into a 50 ml centrifuge tube, and then the cells were discarded.
6. The harvested supernatant was centrifuged at 3500 rpm at room temperature for 10 mins and transferred to a fresh 50 ml centrifuge tube. After centrifugation at 30000 rpm at 4°C for 2 hours in an ultracentrifuge, the supernatant was carefully discarded. The centrifuge tube was inverted and placed on sterilized blotting paper. The precipitate was resuspended in Dulbecco's phosphate-buffered saline (DPBS), then collected into a 1.5 ml EP tube, and stored in a refrigerator at-80°C.

### II. Lentivirus titer test

The method for testing lentivirus titer specifically includes determining lentivirus titer by Real-time quantitative PCR. The specific procedure is as follows.

A sample was prepared as follows. 293T cells were seeded in a 24-well plate at 1×10⁵ cells per well. The lentiviruses were added on day 2, and the medium was replaced with the fresh medium after 12 to 20 hours. At 72 hours after infection, photos were taken for recording the fluorescence. After that, the 293T cells were collected for extraction of genomic DNA, and the titer was tested through quantitative PCR.

The Real-time quantitative PCR was performed on ABI7500 instrument. Reagent SYBR Master Mixture used was from TAKARA.
1. The reaction system was prepared according to the following proportions: SYBR premix ex taq: 10 µl;
   ROX: 0.4 µl;
   Forward primer (25µM): 0.5 µl;
   Reverse primer (25µM): 0.5 µl;
   Genomic DNA: 2.0 µl; and
   water: 6.6 µl.
2. A procedure of two-step Real-time quantification was performed, including pre-denaturation at 95°C for 15 seconds; and denaturation at 95°C for 5 seconds and annealing extension at 60°C for 34 seconds in each cycle, with a total of 40 cycles. The absorbance value was read in each annealing extension stage.
   The PCR procedure was follows:
   Cycle 1: (1×)
   Step 1: 95.0°C for 15 seconds;
   Cycle 2: (40×)
   Step 1: 95.0°C for 5 seconds,
   Step 2: 60.0°C for 34 seconds; and
   initiation of data collection and real-time analysis.
3. A melting curve was prepared. After PCR, denaturation was performed at 95°C for 1 minute, followed by cooling down to 55°C to allow fully binding of the DNA double strands. Then, the reaction temperature was increased from 55°C to 95°C, with an increase of 0.5°C per cycle and each step holding for 30 seconds, as the absorbance value was read.

The procedure for preparing a melting curve was as follows:
Cycle 3: (1×)
Step 1: 95.0 °C for 1 minute;
Cycle 4: (1×)
Step 1: 55.0 °C for 1 minute;
Cycle 5: (81×)
Step 1: 55.0 °C to 95.0 °C, each step holding for 30 seconds,
the set temperature was increased by 0.5°C after every 2 cycles in Cycle 5.

### Example 2. Selection of repressible operon

A total of four transcriptional regulatory systems (i.e., CymR-CuO, tryptophan operon, diphtheria toxin repressor regulatory system, and lactose operon) were tested in the present disclosure.
I. Firstly, cis-acting elements of these four transcriptional regulatory systems were respectively introduced into the lentiviral vectors to test whether the expression of exogenous gene was inhibited in a eukaryotic system.

### 1. CymR-CuO system

The CymR-CuO system is a class of regulatable expression system that function by following principle.

CymR protein can specifically bind to CuO element in the absence of Cumate, thereby inhibiting the gene transcription. In the presence of Cumate, CymR bound to Cumate would detach from the CuO element, thus allowing normal expression of gene.

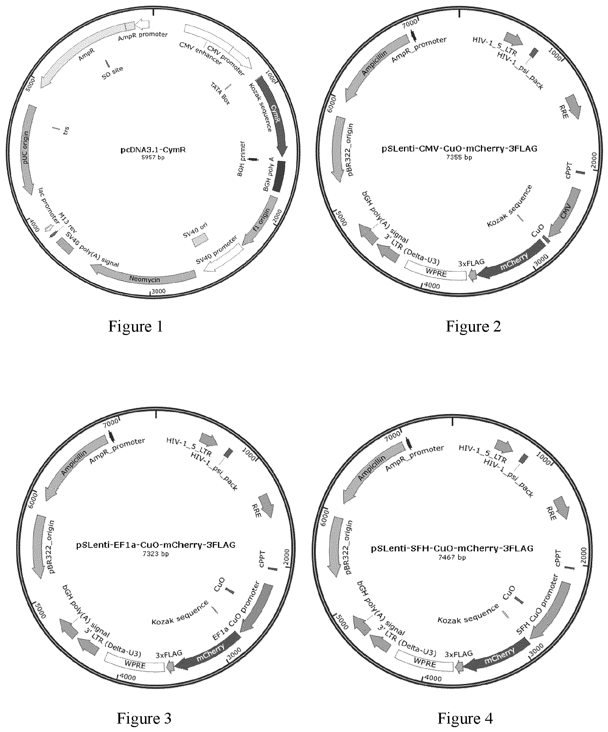

In the present disclosure, a series of promoter vectors CMV-CuO, EF1a-CuO, SFH-CuO and CAG-CuO were constructed. It was confirmed that the CuO element in cooperation with the CymR vector (pcDNA3.1-CymR) can effectively inhibit the gene expression. The plasmid map of pcDNA3.1-CymR is shown in Figure 1. The plasmid maps of CMV-CuO, EF1a-CuO, SFH-CuO, and CAG-CuO promoter vectors are shown in Figure 2 to 5, respectively.

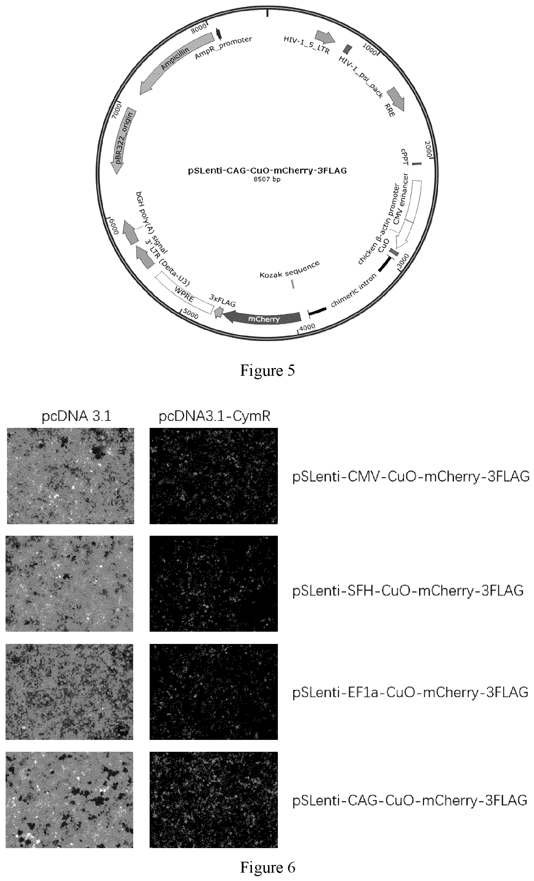

293T cells were co-transfected with the lentiviral vector encoding the CuO element and either pcDNA3.1 empty vector or pcDNA3.1-CymR. Fluorescence photographs were taken after 24 hours. It can be seen that the expression efficiency of CMV-CuO, EF1a-CuO, SFH-CuO and CAG-CuO promoters were significantly inhibited under the action of CymR protein (see Figure 6).

### 2. Tryptophan operon system

In the presence of tryptophan, the Trp repressor (TrpR) protein can specifically bind to the TrpO element and thus inhibits the gene transcription. This tryptophan operon system was found in prokaryotic cells and had long been used as an explanatory demonstration of gene regulation. However, this tryptophan operon system has not been applied in eukaryotic cells because tryptophan is necessary for the culture of mammalian cells.

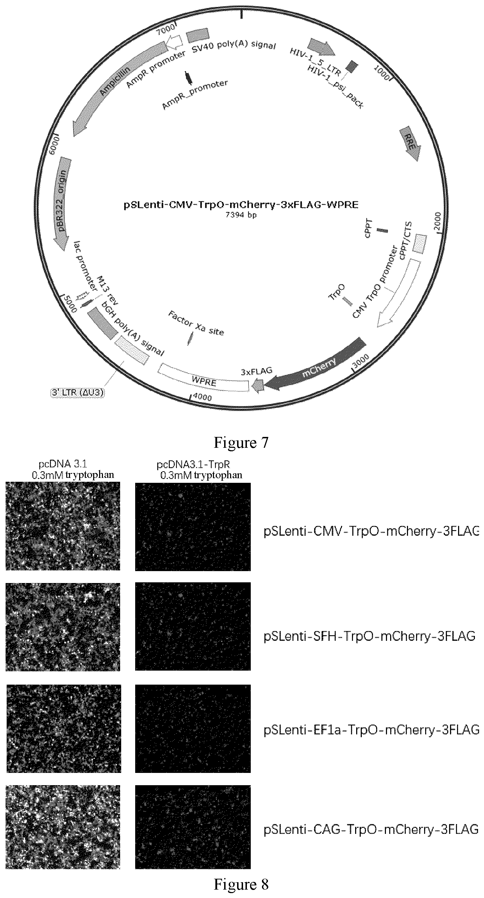

In the present disclosure, a series of promoter vectors such as CMV-TrpO, EF1a-TrpO, SFH-TrpO, CAG-TrpO were constructed. It was confirmed that the TrpO element in cooperation with the TrpR vector (pcDNA3.1-TrpR) can effectively inhibit the gene expression in the presence of tryptophan. The plasmid map of CMV- TrpO is shown in Figure 7. The design of plasmid maps of EF1a-TrpO, SFH-TrpO, and CAG-TrpO promoter vectors can be referred to Figures 2 to 5 on the basis of Figure 7, which will not be repeated.

293T cells were co-transfected with the lentiviral vector encoding the TrpO element and either pcDNA3.1 empty vector or pcDNA3.1-TrpR, with 0.3 mM tryptophan added. Fluorescence photographs were taken after 24 hours. It can be seen that the expression efficiency ofCMV-TrpO, EF1a-TrpO, SFH-TrpO and CAG-TrpO promoters were significantly inhibited under the action of TrpR protein (see Figure 8).

### 3. Diphtheria toxin repressor regulatory system

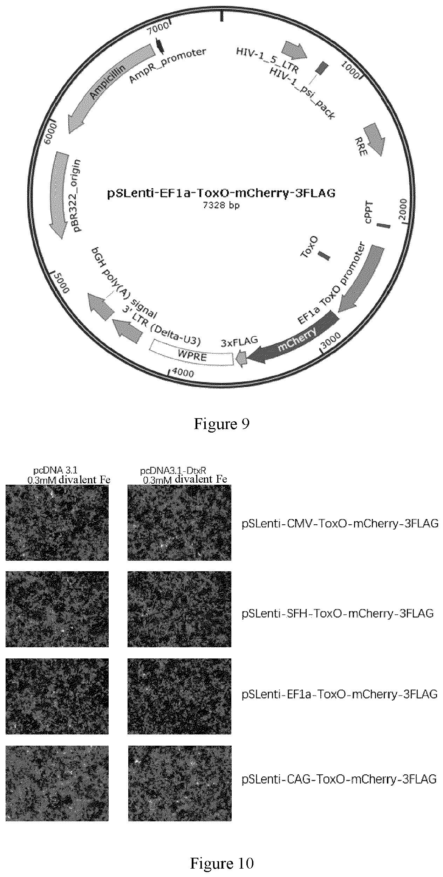

The diphtheria toxin repressor DtxR protein can specifically bind to the ToxO element and thus inhibits the gene transcription. In the present disclosure, a series of promoter vectors such as CMV-ToxO, EF1a-ToxO, SFH-ToxO, CAG-ToxO were constructed. 293T cells were co-transfected with the lentiviral vector encoding the ToxO element and either pcDNA3.1 empty vector or pcDNA3.1-DtxR, with divalent iron ions added. As a result, the gene of interest was not found to be significantly inhibited (see Figure 10).

The plasmid map of EF1a- ToxO is shown in Figure 9. The design of plasmid maps of CMV-ToxO, SFH-ToxO and CAG-ToxO promoter vectors can be referred to Figures 2 to 5 on the basis of Figure 9, which will not be repeated.

### 4. lactose operon system

The lactose operon repressor LacI protein can specifically bind to the LacO element and thus inhibits the gene transcription. In the presence of isopropyl β-D-1-thiogalactopyranoside (IPTG), the LacI protein binds to the IPTG and is subjected to conformational change, thereby losing its binding ability and allowing the expression of downstream gene. The lactose operon system is widely used in prokaryotic cells for inducing the expression of exogenous genes.

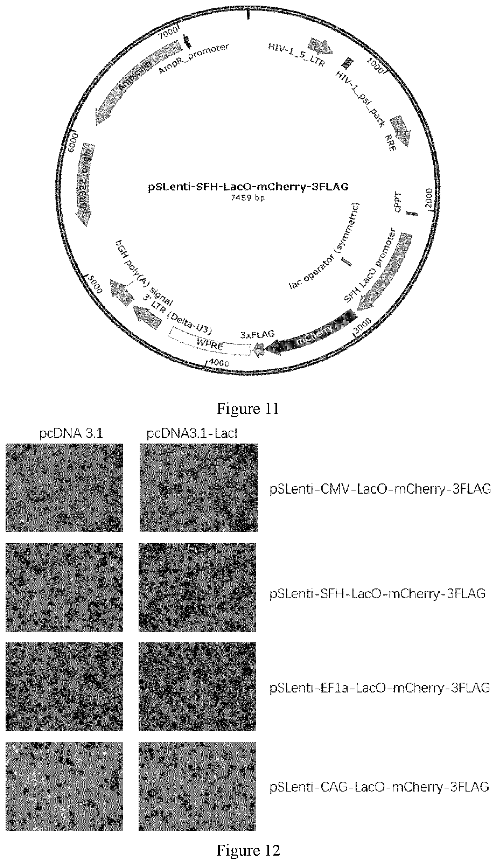

In the present disclosure, a series of promoter vectors such as CMV-LacO, EF1a-LacO, SFH-LacO, CAG-LacO were constructed. 293T cells were co-transfected with the lentiviral vector encoding the LacO element and either pcDNA3.1 empty vector or pcDNA3.1-LacI. As a result, the gene of interest was not found to be significantly inhibited (see Figure 12).

The plasmid map of SFH-LacO is shown in Figure 11. The design of plasmid maps of CMV-LacO, EF1a-LacO, and CAG-LacO promoter vectors can be referred to Figures 2 to 5 on the basis of Figure 11, which will not be repeated.

### II. Optimization of insertion sites of repressible operon elements

### 1. TrpO element

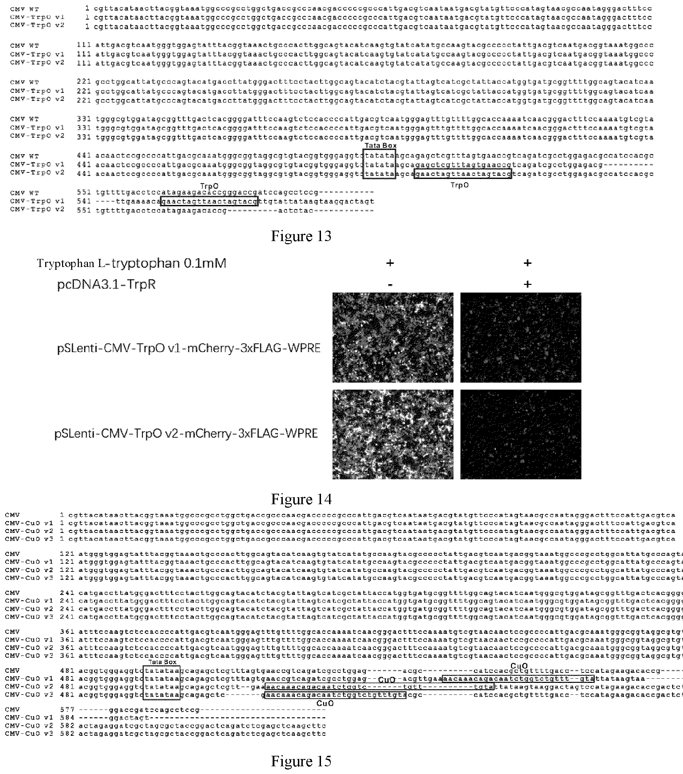

The distance between the TrpO element and the TATA Box of CMV can vary. The inventors have designed two promoters as shown in Figure 13. At 48 hours after co-transfection with the plasmid encoding the TrpO element and pcDNA3.1-TrpR (with 0.1mM tryptophan added), the fluorescence was observed. The experiment shows that varying the distance between the TrpO element and the TATA Box can result in different inhibitory effects (see Figure 14). The TrpO v2 promoter sequence with a better inhibition effect was used in subsequent steps in the present disclosure.

### 2. CuO element

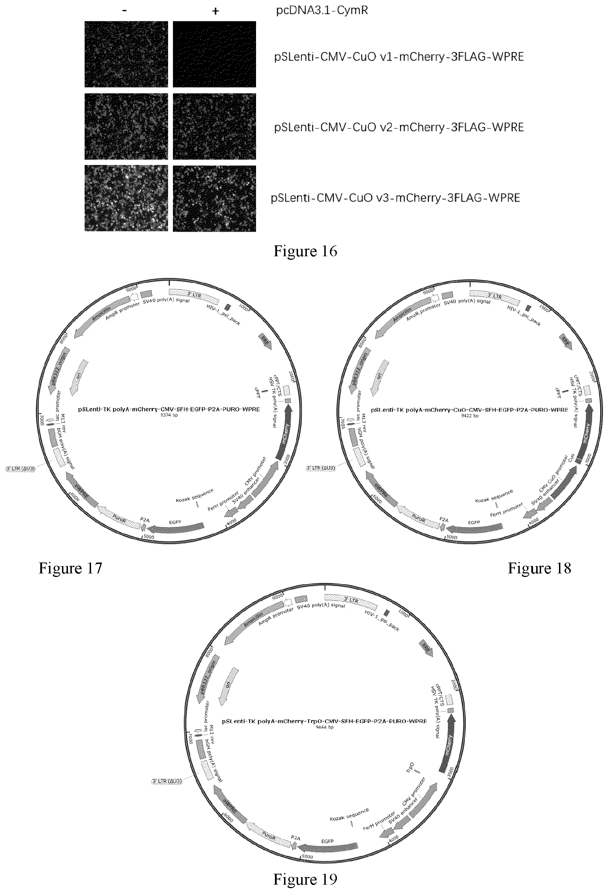

The distance between the CuO element and the TATA Box of CMV can vary. The inventors have designed three promoters, as shown in Figure 15. At 48 hours after co-transfection with the plasmid encoding the CuO element and pcDNA3.1-CymR, the fluorescence was observed. The experiment shows that varying the distance between the CuO element and the TATA Box can result in different inhibitory effects (see Figure 16). The CuO v1 promoter sequence with a better inhibition effect was used in subsequent steps in the present disclosure.

### Example 3. Increased titer of virus expressed from lentiviral vector including reverse expression cassette by repressible operon

In the case where a reverse expression cassette is included in the lentivirus, the viral RNA is hardly transcribed due to the presence of the reverse expression cassette, so the virus titer is very low.

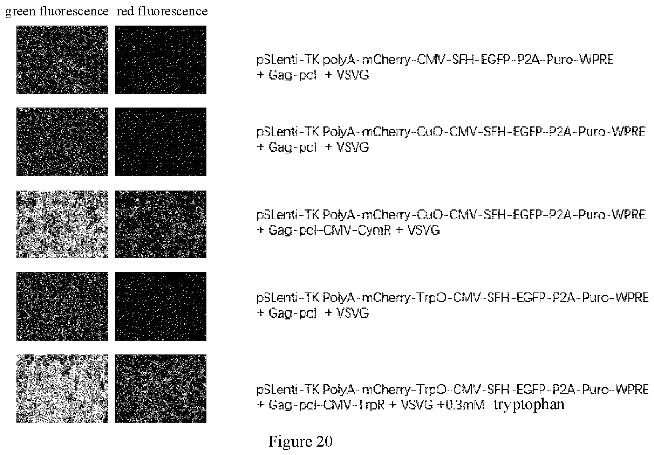

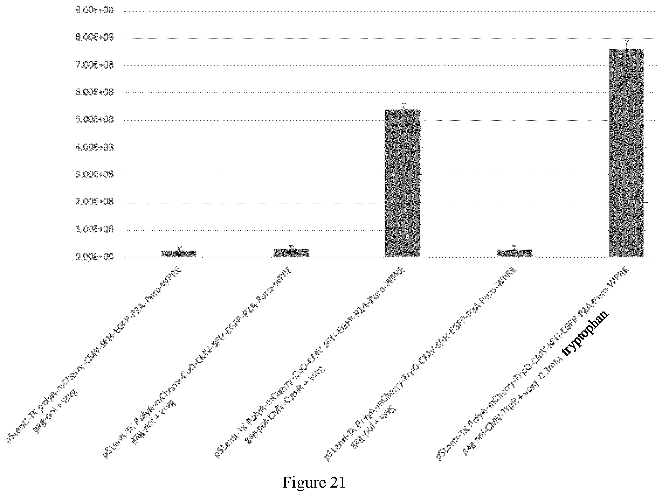

In this example, vectors pSLenti-TK polyA-mCherry-CMV-SFH-EGFP-P2A-Puro-WPRE (Figure 17), pSLenti-TK polyA-mCherry-CuO-CMV-SFH-EGFP-P2A-Puro-WPRE (Figure 18), and pSLenti-TK polyA-mCherry-TrpO-CMV-SFH-EGFP-P2A-Puro-WPRE (Figure 19) were respectively constructed. The problem of low virus release due to the presence of the reverse expression cassette can be effectively solved by inhibition of the expression of the protein by using reverse expression cassettes containing CMV-CuO, or CMV-TrpO promoter, in combination with CymR, or TrpR plus tryptophan in lentiviral packaging, respectively. As results of the experiments, the fluorescence images are shown in Figure 20, and the statistics diagram of lentivirus titer is shown in Figure 21.

This mode for expression is very suitable for the cases of overexpression of LncRNA, miRNA, circRNA and other genes.

### Example 4 Influence of different polyA signal sequences in lentiviral vectors including reverse expression cassettes on titer

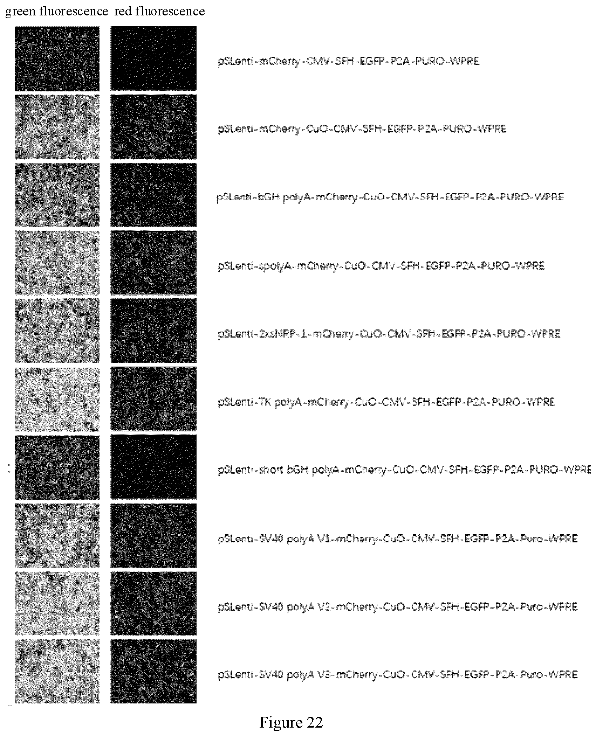

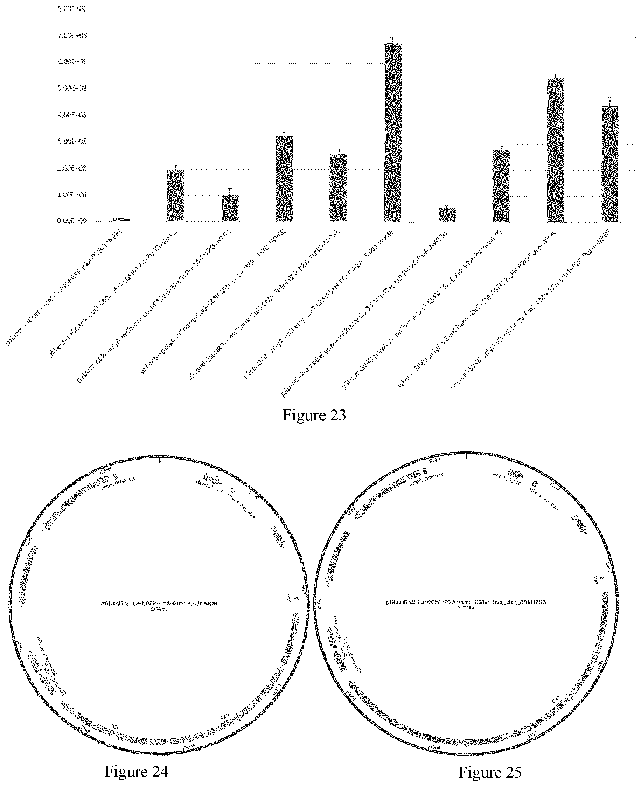

According to the experimental results, in the lentiviral vector including the reverse expression cassette, the reverse gene can be well expressed even in the absence of reverse polyA signal. It was speculated that the tttatt sequence downstream of the cPPT sequence in the lentiviral vector may play the role of polyA signal. In spite of this, it is of great importance to incorporate a reverse polyA which does not affect the titer so as to timely terminate the transcription of the reverse expression cassette because the purpose of the reverse expression cassette is to control the transcription more precisely. With the CuO-cumate system used, each of eight different polyA signals was positioned in the lentiviral vectors including reverse expression cassettes. Except for bGH polyA and short bGH polyA which have a greater impact on the lentivirus titer, titers of the viruses expressed from other vectors are all greater than or equal to those expressed from of lentiviral vector including a reverse expression cassette without polyA. TK polyA and SV40 polyA V2 perform the best, rendering greater virus titers to the lentiviral vectors including the reverse expression cassette. As results of the experiments, the fluorescence images are shown in Figure 22. The statistics diagram of lentiviral titer is shown in Figure23.

### Example 5 Expression of hsa_circ_0008285 gene from lentiviral vector including reverse expression cassette

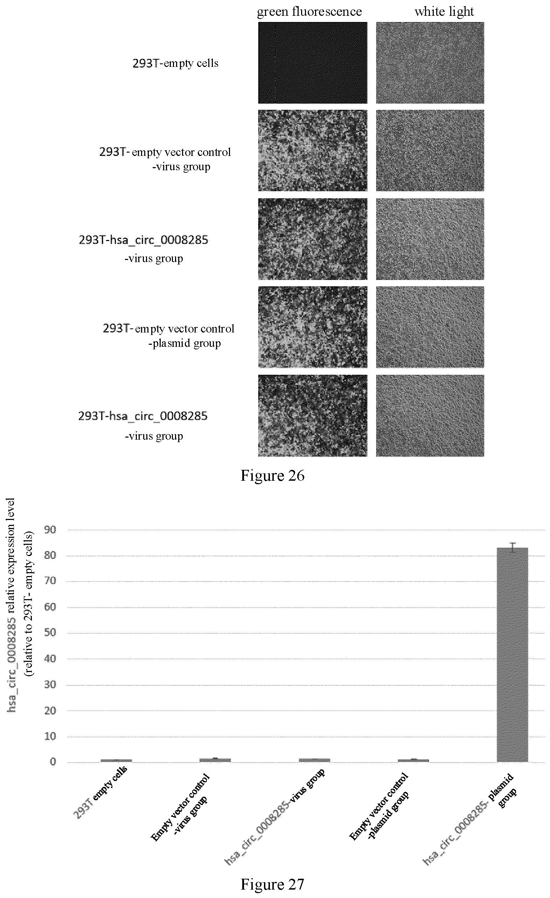

In the virus production, it was found that in the case where hsa_circ_0008285 gene was incorporated into a common lentiviral vector, normal expression of the hsa_circ_0008285 gene was not detectable in the obtained lentiviral particles. Specifically, based on the empty vector pSLenti-EF1a-EGFP-P2A-Puro-CMV-MCS (vector map as shown in Figure 24), the inventors constructed a vector pSLenti-EF1a-EGFP-P2A-Puro-CMV- hsa_circ_0008285 (vector map as shown in Figure 25), including the hsa_circ_0008285 gene inserted forward and no repressible operon. The empty vector pSLenti-EF1a-EGFP-P2A-Puro-CMV-MCS and the vector pSLenti-EF1a-EGFP-P2A-Puro-CMV- hsa_circ_0008285 were each used to package lentivirus. After titer tests of the packaged lentiviruses, 293T cells were infected with the packaged lentiviruses each at the multiplicity of infection (MOI) of 5. At the same time, the plasmids pSLenti-EF1a-EGFP-P2A-Puro-CMV-MCS and pSLenti-EF1a-EGFP-P2A-Puro-CMV- hsa_circ_0008285 were each transfected into 293T cells using lipo2000. The groups include as follows: empty vector-virus group (i.e., virus empty control group), hsa_circ_0008285-virus group (i.e., virus expression group), empty vector-plasmid group (i.e., plasmid empty control group) and hsa_circ_0008285-plasmid group (i.e., plasmid expression group). 48 hours after infection or transfection, fluorescent pictures were taken with a fluorescence microscope. The results of the fluorescent pictures are shown in Figure 26. For the virus infection groups and plasmid transfection groups of pSLenti-EF1a-EGFP-P2A-Puro-CMV-MCS and pSLenti-EF1a-EGFP-P2A-Puro-CMV-hsa_circ_0008285, green fluorescence was normally expressed in the groups. 48 hours after infection or transfection, the cells were collected, and the expression of hsa_circ_0008285 was tested by QPCR method. The results are shown in Figure 27. The results of QPCR showed that the expression of hsa_circ_0008285 was only detected in the plasmid expression groups, and its expression level in the plasmid expression groups was about 83 times higher than that in the control group. However, for the virus infection group of pSLenti-EF1a-EGFP-P2A-Puro-CMV- hsa_circ_0008285, the expression of hsa_circ_0008285 was not detected. This indicated that the lentiviral particles obtained by incorporating the hsa_circ_0008285 gene into a common lentiviral vector cannot normally express the hsa_circ_0008285 gene.

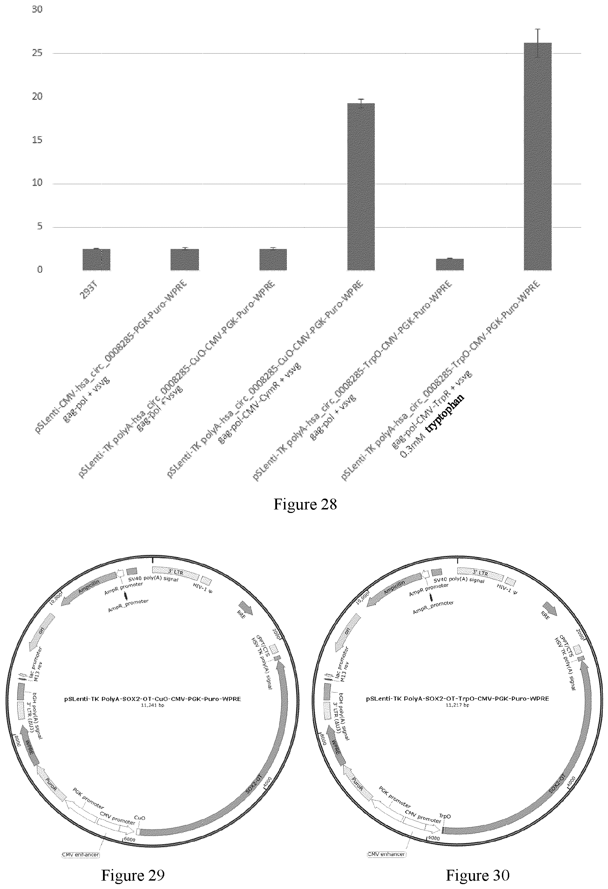

Further, in this example, by using reverse expression cassettes including CMV-CuO promoter or CMV-TrpO promoter, vectors pSLenti-TK polyA-hsa_circ_0008285-CMV-PGK-Puro-WPRE, pSLenti-TK polyA-hsa_circ_0008285-CuO-CMV-PGK-Puro-WPRE, and pSLenti-TK polyA-hsa_circ_0008285-TrpO-CMV-PGK-Puro-WPRE were respectively constructed. The vectors are used in combination with CymR, or TrpR plus tryptophan to inhibit the expression of protein in the lentiviral packaging. After infection of 293T cells with the packaged lentivirus, the expression of hsa_circ_0008285 gene was tested by qPCR. The results showed that the group of pSLenti-TK poly A-hsa_circ_0008285-CuO-CMV-PGK-Puro-WPRE/CymR, and the group of pSLenti-TK polyA-hsa_circ_0008285-TrpO-CMV-PGK-Puro-WPRE/TrpR and tryptophan both exhibited an expression level of hsa_circ_0008285 gene significantly higher than that in the control group, confirming that the lentiviral vectors including reverse expression cassettes of CMV-CuO promoter or CMV-TrpO promoter can effectively achieve the overexpression of the hsa_circ_0008285 gene. The experimental results are shown in Figure 28.

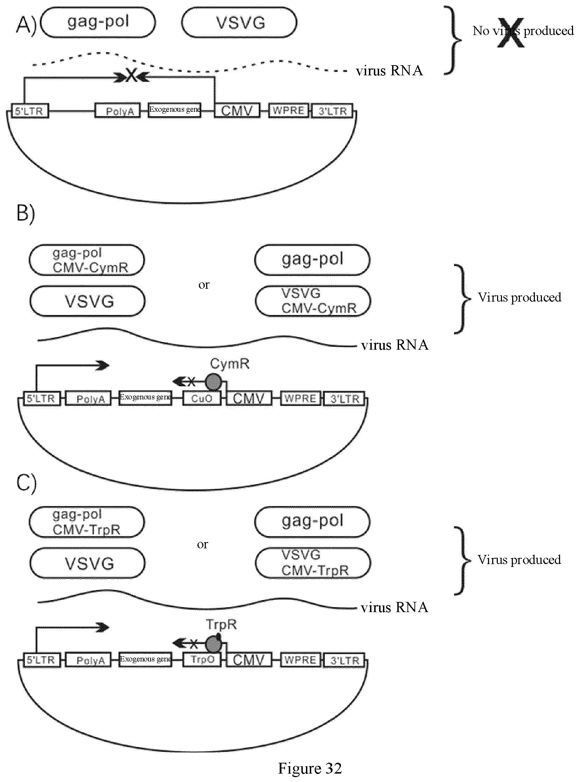

It can be seen from the figures that almost no expression is detectable in vectors pSLenti-TK polyA-hsa_circ_0008285-CuO-CNW-PGK-Puro-WPRE or pSLenti-TK polyA-hsa_circ_0008285-TrpO-CMV-PGK-Puro-WPRE in the absence of CymR repressor or tryptophan. The vector pSLenti-TK polyA-hsa_circ_0008285-CMV-PGK-Puro-WPRE including no repressible operon but the reversely inserted expression cassette still could not provide normal packaging, indicating that expression of the reversely inserted expression cassette could affect the normal expression of the gene of interest. However, the two groups containing both the reversely inserted expression cassette polyA-hsa_circ_0008285-CuO and the repressor can provide normal expression This shows that the reversely inserted expression cassette in cooperation with the repressible operon and repressor can realize the expression of hsa_circ_0008285. For its principle, see the illustration in Figure 32.

### Example 6 Expression of SOX2-OT gene by lentiviral vector including reverse expression cassette

In the virus production, it was found that in the case where SOX2-OT gene was incorporated into a common lentiviral vector, normal expression of the SOX2-OT gene was not detectable in the obtained lentiviral particles. Specifically, based on the empty vector pSLenti-EF1a-EGFP-P2A-Puro-CMV-MCS (vector map as shown in Figure 24), the inventors constructed a vector pSLenti-EF1a-EGFP-P2A-Puro-CMV- SOX2-OT (vector map as shown in Figure 24), including the SOX2-OT gene inserted forward and no repressible operon. The empty vector pSLenti-EF1a-EGFP-P2A-Puro-CMV-MCS and the vector pSLenti-EF1a-EGFP-P2A-Puro-CMV- SOX2-OT were each used to package lentivirus. As results of titer tests of the packaged lentiviruses, the titer of the empty vector pSLenti-EF1a-EGFP-P2A-Puro-CMV-MCS was 2.13E+8 TU/ml, while the titer of the vector pSLenti-EF1a-EGFP-P2A-Puro-CMV- SOX2-OT was 1.81E+7 TU/ml, indicating that the virus could not be normally packaged and released when the SOX2-OT gene was incorportated into a common lentiviral vector.

Further, in this example, by using reverse expression cassettes including CMV-CuO promoter or CMV-TrpO promoter, vectors pSLenti-TK poly A- SOX2-OT-CMV-PGK-Puro-WPRE, pSLenti-TK polyA-SOX2-OT-CuO-CMV-PGK-Puro-WPRE (in which its vector map is shown in Figure 29), and pSLenti-TK poly A- SOX2-OT-TrpO-CMV-PGK-Puro-WPRE (in which its vector map is shown in Figure 30) were respectively constructed. The vectors are used in combination with CymR, or TrpR plus tryptophan to inhibit the expression of protein in the lentiviral packaging. As results of titer test of the packaged lentiviruses, the titer of the empty vector pSLenti-TK poly A- SOX2-OT-CMV-PGK-Puro-WPRE was 1.17E+7 TU/ml; the titer of the group pSLenti-TK polyA-SOX2-OT-CuO-CMV-PGK-Puro-WPRE/CymR was 4.44E+8 TU/ml; and the titer of the group pSLenti-TK polyA-SOX2-OT-TrpO-CMV-PGK-Puro-WPRE/TrpR and tryptophan was 2.58E+8 TU/ml. These indicate that the virus could not be packaged and released normally using the vector (pSLenti-TK poly A- SOX2-OT-CMV-PGK-Puro-WPRE) including no repressible operon but the reversely inserted expression cassette. At the same time, the virus could not be packaged and released normally using vectors pSLenti-TK poly A- SOX2-OT-CuO-CMV-PGK-Puro-WPRE or pSLenti-TK poly A- SOX2-OT-TrpO-CMV-PGK-Puro-WPRE in the absence of the CymR repressor or tryptophan. However, using the lentiviral vectors including reverse expression cassettes of CMV-CuO promoter or CMV-TrpO promoter the normal packaging from SOX2-OT gene vectors and the viral release could be successfully achieved.

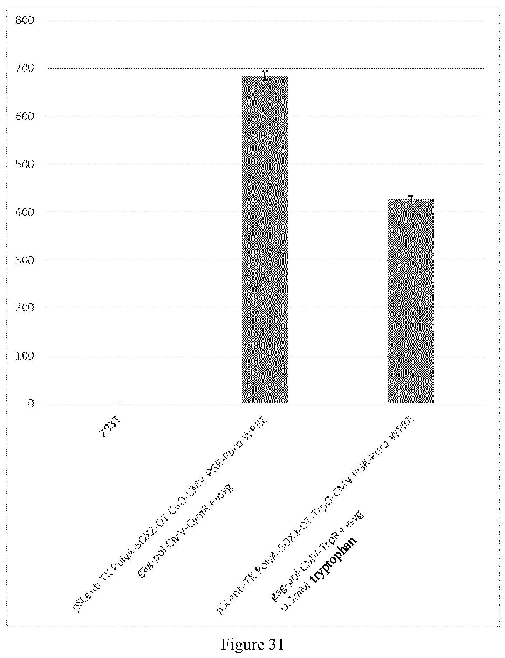

After infection of 293T cells with the packaged lentivirus, 293T cells were respectively infected at the multiplicity of infection (MOI) of 5. 48 hours after infection, the expression of SOX2-OT gene was tested by qPCR. The results showed that the expression of SOX2-OT gene in the group pSLenti-TK polyA-SOX2-OT-CuO-CMV-PGK-Puro-WPRE/CymR and the group pSLenti-TK polyA-SOX2-OT-TrpO-CMV-PGK-Puro-WPRE/TrpR plus tryptophan was significantly higher than that in the control group (293T), confirming that the lentiviral vectors including reverse expression cassettes of CMV-CuO promoter or CMV-TrpO promoter can effectively provide the overexpression of the SOX2-OT gene. The experimental results are shown in Figure 31. This shows that the reversely inserted expression cassette in cooperation with the repressible operon and repressor can realize the packaging of SOX2-OT, the viral release, and the gene expression. For its principle, see the illustration in Figure 32.

The technical features of the examples described above can be combined arbitrarily. For the sake of conciseness, all possible combinations of the technical features of the above examples are not described. However, as long as these combinations of the technical features do not contradict each other, they should be considered to be within the scope of this specification.

The examples described above in a specific and detailed manner merely express several embodiments of the present disclosure. However, they should not be construed as a limitation to the scope of the present disclosure. It should be noted that a number of variations and improvements can be made for a person of ordinary skill in the art without departing from the conception of the present disclosure, all of which fall within the protection scope of the present disclosure. Therefore, the protection scope of the present disclosure shall be subject to the appended claims.

## Claims

1. A vector for lentiviral packaging, comprising a first long terminal repeat, a reversely inserted gene expression cassette, and a second long terminal repeat,
wherein the first long terminal repeat is positioned upstream of the reversely inserted gene expression cassette in a direction of viral genome expression,
the second long terminal repeat is positioned downstream of the reversely inserted gene expression cassette in a direction of viral genome expression,
the reversely inserted gene expression cassette comprises a promoter, a repressible operon, a gene of interest, and a polyadenylation signal which are linked in sequence,
the repressible operon represses expression of the gene of interest in the presence of a repressor, and
the gene of interest is a gene whose expression needs to be precisely terminated.

2. The vector for lentiviral packaging according to claim 1, wherein the gene of interest is used to express non-coding RNA expressed by Pol II promoter.

3. The vector for lentiviral packaging according to claim 2, wherein the non-coding RNA is selected from snRNA, lncRNA, amiRNA, or circ-RNA.

4. The vector for lentiviral packaging according to claim 1, wherein the polyadenylation signal is TK polyA and/or SV40 polyA V2.

5. The vector for lentiviral packaging according to any one of claims 1 to 4, wherein the repressible operon is selected from a tryptophan operon and/or a Cumate-CuO regulable system.

6. The vector for lentiviral packaging according to claim 5, wherein a distance between a site where a TrpO element of a tryptophan operon is inserted into the gene expression cassette and a TATA BOX of the promoter is less than or equal to 18 nucleotides.

7. The vector for lentiviral packaging according to claim 6, wherein a distance between the site where a TrpO element of a tryptophan operon is inserted into the gene expression cassette and the TATA BOX of the promoter is greater than 8 nucleotides.

8. The vector for lentiviral packaging according to claim 5, wherein a distance between a site where a CuO element of a Cumate-CuO regulable system is inserted and a TATA BOX of the promoter is 40 to 50 nucleotides.

9. The vector for lentiviral packaging according to any one of claims 1 to 4 and 6 to 8, wherein the promoter is a CMV, EF1a, SFH, CAG, CBh, UBC, SFFV, SV40, RSV, mCMV, GAPDH, PGK, CASI, SMVP, GUSB, or UCOE promoter.

10. The vector for lentiviral packaging according to any one of claims 1 to 4 and 6 to 8, wherein the vector for lentiviral packaging further comprises at least one of a reporter gene, an enhancer, an internal ribosome entry site, or a terminator.

11. A lentiviral vector packaging system, comprising the vector for lentiviral packaging of any one of claims 1 to 10, wherein the lentiviral vector packaging system is capable of producing HIV vector particles having only primary infectivity and no replication capacity.

12. The lentiviral vector packaging system according to claim 11, wherein the lentiviral vector packaging system is a two-plasmid packaging system, a three-plasmid packaging system, or a four-plasmid packaging system.

13. A method for packaging a lentivirus, comprising:
transferring the lentiviral vector packaging system of claim 11 or 12 into a host cell, and
performing packaging in the presence of a repressor.
